# EUROPÄISCHE PATENTANMELDUNG

(11) **EP 0 586 866 A2**
(43) Veröffentlichungstag der Anmeldung: **16.03.1994**
(21) Anmeldenummer: 93112134.7
(22) Anmeldetag: 29.07.1993
(51) Int. Cl.: C07D 405/12, C07D 405/14, A61K 31/44

(54) **1,4-Benzodioxanderivate mit pharmakologischen Eigenschaften**

(30) Priorität: 11.08.1992 DE 4226527
(71) Anmelder: MERCK PATENT GmbH, D-64293 Darmstadt (DE)
(72) Erfinder: Böttcher, Henning, Dr., D-64283 Darmstadt (DE); Seyfried, Christoph, Dr., D-64342 Seeheim-Jugenheim (DE); Greiner, Hartmut, Dr., D-64287 Darmstadt (DE); Bartoszyk, Gerd, D-64289 Darmstadt (DE)

(57) **Zusammenfassung**

1,4-Benzodioxanderivate der Formel I 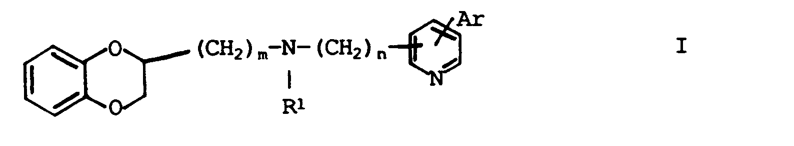 worin
R¹ H oder A,
Ar einen unsubstituierten oder einen ein- oder zweifach durch A, F, Cl, Br, I, CN, OH und/oder OA oder durch eine Methylendioxygruppe substituierten Phenylrest,
A Alkyl mit 1-6 C-Atomen und
m und n jeweils unabhängig voneinander 1 oder 2

bedeuten, sowie deren Salze zeigen Wirkungen auf das Zentralnervensystem.

## Beschreibung

Die Erfindung betrifft 1,4-Benzodioxanderivate der Formel I 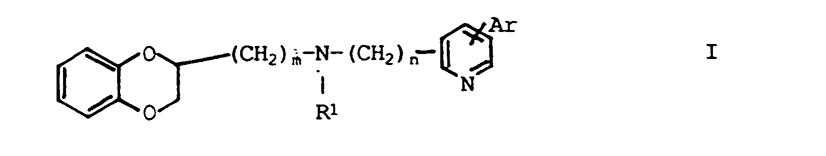 worin
R¹ H oder A,
Ar einen unsubstituierten oder einen ein- oder zweifach durch A, F, Cl, Br, I, CN, OH und/oder OA oder durch eine Methylendioxygruppe substituierten Phenylrest,
A Alkyl mit 1-6 C-Atomen
und
m und n jeweils unabhängig voneinander 1 oder 2
bedeuten, sowie deren Salze und deren im Stoffwechsel auftretende Metaboliten.

Ähnliche Verbindungen werden in DE 36 09 142 beschrieben.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N.raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-64 8) der direkt gemessene Blutdruck nach peroraler Gabe der Verbindungen gesenkt. Ebenso eignen sie sich zur Prophylaxe und zur Bekämpfung der Folgen cerebraler Infarktgeschehen (Apoplexia cerebri), wie Schlaganfall und cerebrale Ischämien.

Verbindungen der Formel I und ihre physiologisch unbedenklichen Säureadditionssalze können daher als Arzneimittelwirkstoffe für Anxiolytika, Antidepressiva, Neuroleptika und/oder Antihypertonika und auch als Zwischenprodukte zur Herstellung anderer Arzneimittelwirkstoffe verwendet werden.

Gegenstand der Erfindung sind die 1,4-Benzodioxanderivate der Formel I sowie ihre physiologisch unbedenklichen Säureadditionssalze und ihre im Stoffwechsel auftretenden Metaboliten.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy.

Der Rest Ar bedeutet vorzugsweise unsubstituiertes Phenyl, aber auch ein- oder zweifach substituiertes Phenyl. Falls der Phenylrest zweifach substituiert ist, können die Substituenten gleich oder verschieden sein. Bevorzugte Substituenten an der Phenylgruppe sind F, Cl, Methoxy, CN oder Methyl. Die Substituenten befinden sich in ortho-, meta- und/oder para-Position, wobei zweifach substituierte Phenylreste bevorzugt ortho- und para-substituiert sind. Im einzelnen ist Ar bevorzugt Phenyl, o-, m- oder p-Trifluormethylphenyl, o-, m- oder p-Methoxyphenyl, o-, m- oder p-Fluorphenyl, o-, m- oder p-Methylphenyl, o-, m- oder p-Cyanphenyl oder 2,4-Dimethoxyphenyl, aber auch o-, m- oder p-Ethoxyphenyl, o-, m- oder p-Bromphenyl, 2,3-, 2,5-, 2,6-, 3,4- oder 3,5-Dimethoxyphenyl, 2,3- oder 3,4-Methylendioxyphenyl.

Die Parameter m und n bedeuten jeweils unabhängig voneinander 1 oder 2, vorzugsweise jeweils 1.

R¹ steht besonders bevorzugt für Methyl, ferner aber auch für Ethyl, Propyl, Isopropyl, n-Butyl, sek.-Butyl oder tert.-Butyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgende Teilformeln la bis Ig ausgedrückt werden, die der Formel I entsprechen und worin die nicht näher bezeichneten Reste und Parameter die bei der Formel I angegebene Bedeutung haben, worin jedoch
in la Ar p-Fluorphenyl und m und n jeweils 1 bedeuten;
in Ib Ar Phenyl und m und n jeweils 1 bedeuten;
in Ic Ar p-Methoxyphenyl und m und n jeweils 1 bedeuten;
in Id Ar Phenyl bedeutet und mit der 4-Position des Pyridyl-Restes verknüpft ist;
in le Ar Phenyl, p-Methoxyphenyl oder p-Fluorphenyl bedeutet und mit der 5-Position des Pyridyl-Restes verknüpft ist;
in If Ar Phenyl, p-Methoxyphenyl oder p-Fluorphenyl und R¹ H bedeuten;
in Ig Ar Phenyl, p-Methoxyphenyl oder p-Fluorphenyl und R¹ Methyl bedeuten.

Gegenstand der Erfindung ist ferner ein Verfahren zur Herstellung von 1,4-Benzodioxanderivaten der Formel I sowie von deren Salzen, dadurch gekennzeichnet, daß man eine Verbindung der Formel II 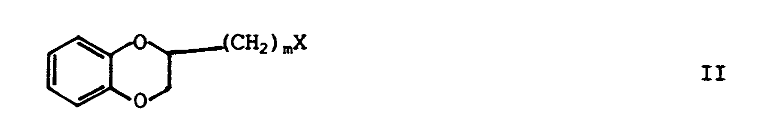 worin m die angegebene Bedeutung hat und
X Cl, Br, I, OH oder eine reaktionsfähige, funktionell abgewandelte OH-Gruppe bedeutet, mit einer Verbindung der Formel III 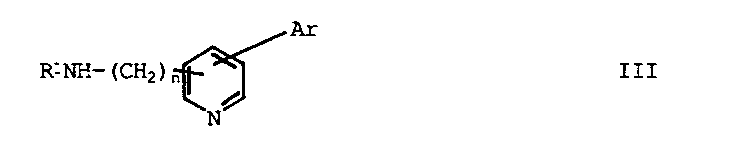 worin
R¹, Ar und n die angegebenen Bedeutungen haben, umsetzt, oder daß man eine Verbindung der Formel IV 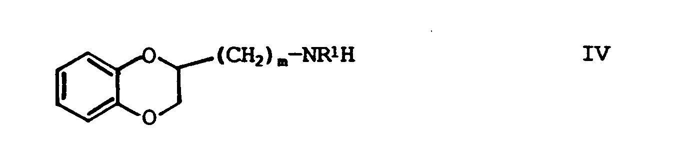 worin R¹ und m die angegebenen Bedeutungen haben, mit einer Verbindung der Formel V 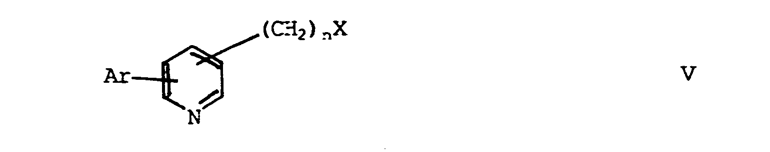 worin
Ar, n und X die angegebenen Bedeutungen haben, umsetzt, oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzlich C-C-und/oder C-N-Bindung(en) enthält, mit einem reduzierenden Mittel behandelt, oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls ein sekundäres Amin der Formel I (R¹ = H) durch Alkylierung in ein tertiäres Amin der Formel I (R¹ = A) umwandelt und/oder daß man eine Gruppe Ar in eine andere Gruppe Ar und/oder daß man eine erhaltene Base der Formel I durch Behandeln mit einer Säure in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 36 09 142) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Es ist möglich eine Verbindung der Formel I durch Umsetzung einer Verbindung der Formel 11 mit einer Verbindung der Formel III zu erhalten.

In den 1,4-Benzodioxanderivaten der Formel 11 bedeutet X vorzugsweise CI oder Br; es kann jedoch auch I, OH oder eine reaktionsfähige, funktionell abgewandelte OH-Gruppe bedeuten, insbesondere Alkylsulfonyloxy mit 1-6 C-Atomen, beispielsweise Methansulfonyloxy oder Arylsulfonyloxy mit 6-10 C-Atomen, z.B. Benzolsulfonyloxy, p-Toluolsulfonyloxy oder 1- oder 2-Naphthalinsulfonyloxy. Der Parameter m ist vorzugsweise 1.

In Verbindungen der Formel III bedeutet R¹ vorzugsweise H oder Methyl, n bevorzugt 1 und Ar vorzugsweise unsubstituiertes oder durch F, Cl,Br, Methyl oder Methoxy substituiertes Phenyl.

Die Verbindungen der Formel 11 und III sind zum Zeil bekannt; die nicht bekannten Verbindungen der Formeln II und III können leicht in Analogie zu den bekannten Verbindungen hergestellt werden.

So ist es beispielsweise möglich, Verbindungen der Formel II durch Reduktion von Benzodioxanen, die in 2-Stellung eine COOH-, COOA-, -CH₂COOH- oder -CH₂COOA-Gruppe tragen, zu erhalten.

Zur Herstellung der Verbindungen der Formel III können beispielsweise die an sich bekannten Methoden zur Darstellung von primären und sekundären Aminen verwendet werden.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente der Formel III kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, eine Verbindung der Formel I zu erhalten, indem man eine Verbindung der Formel IV mit einem Pyridinderivat der Formel V umsetzt.

Die Verbindungen der Formel IV sind zum Teil bekannt; die nicht bekannten Verbindungen können leicht in Analogie zu den bekannten hergestellt werden. So lassen sich Verbindungen der Formel IV (R1 = H) leicht durch Reduktion von entsprechenden Nitrilen herstellen. Ferner ist es möglich, durch Umsetzung von Brenzcatechin oder dessen reaktionsfähigen Derivaten, mit geschützten 1-Amino-2,3-dihalogenpropan- derivaten und nachfolgende Abspaltung der Aminoschutzgruppe Verbindungen der Formel IV zu erhalten.

Die Verbindungen der Formel V könne durch Reduktion von entsprechenden Pyridincarbonsäuren, Pyridincarbonsäureestern, Pyridinessigsäuren oder Pyridinessigsäureestern, erhalten werden.

Die Umsetzung der Verbindungen IV und V verläuft in der Regel nach den gleichen Methoden, wie sie oben für die Umsetzung von II mit III genannt sind.

Eine weitere Möglichkeit zur Herstellung von Verbindungen der Formel I besteht darin, daß man eine Verbindung, die an sich der Formel I entspricht, aber anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250_{°} in Gegenwart mindestens eines inerten Lösungsmittels.

Reduzierbare (durch Wasserstoff ersetzbare)Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I zu überführen. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall ein einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAIH₄, NaBH₄, Diisobutylaluminiumhydridoder NaAI(OCH₂CH₂0CH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 2500. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200 zersetzt. Die Wolff-Kishner-Reduktion kann auch bei Raumtemperatur in Dimethylsulfoxid mit Hydrazin ausgeführt werden.

Darüber hinaus ist es möglich, Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder auch OH-Gruppen durch Wasserstoff ersetzen.

Verbindungen, die sonst der Formel I entsprechen, aber anstelle eines oder mehrerer H-Atome eine oder mehrere solvolysierbare Gruppe (n) enthalten, können zu den Verbindungen der Formel I solvolysiert, insbesondere hydrolysiert werden.

Die Ausgangsstoffe für die Solvolyse sind beispielsweise Verbindungen, die der Formel I entsprechen, aber anstelle eines H-Atoms am sekundären N-Atom eine solvolysierbare Gruppe enthalten. So können insbesondere Acylderivate (entsprechend der Formel I, aber eine Acylgruppe anstelle von R¹, vorzugsweise eine Alkanoyl-, Alkylsulfonyl- oder Arylsulfonylgruppe mit jeweils bis zu 10 C-Atomen, wie Methan-, Benzol-oder p-Toluolsulfonyl) zu den entsprechenden Verbindungen der Formel I, worin R¹ = H ist, hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel 1 nach an sich bekannten Methoden in eine andere Verbindung der Formel I umwandeln.

So können beispielsweise Verbindungen der Formel I, worin R¹ = H ist, durch Umsetzung mit Alkylhalogeniden alkyliert werden. Die Beeingungen für derartige Reaktionen sind in der Regel bekannt und in den Standardwerken der chemischen Literatur beschrieben (z.B. J. March, Adv. Org. Chem. 3rd Ed., J. Wiley % Sons (1985)).

Desweiteren ist es möglich, einen Rest Ar in einen anderen Rest Ar umzuwandeln.

Umgekehrt kann man eine OH-Gruppe nach an sich bekannten Alkylierungsmethoden in eine OA-Gruppe umwandeln.

Verbindungen der Formel I, die eine OA-Gruppe enthalten, können durch Etherspaltung in die entsprechenden Hydroxyderivate überführt werden. Z.B. kann man die Ether spalten durch Behandeln mit Diisobutylaluminiumhydrid (DIBAH) oder Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-2500.

Eine erhaltene Base der Formel 1 kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropions- äure, Citronensäure, Gluconsäure, Ascorbinsäure, Nicotinsäure,lsonicotinsäure' Methan-oder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäuren, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Gegenstand der Erfindung ist ferner die Verwendung der Verbindungen der Formel I und ihrer physiologisch ubbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen und/oder Psychosen und von Nebenwirkungen bei der Behandlung der Hypertonie (z.B. mit a-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Sämtliche Temperaturen sind in _{°} C angegeben. die Rf-Werte wurden dünnschichtchromatographisch an Kieselgel bestimmt. Das verwendete Laufmittel wird zusammen mit dem jeweiligen Wert angegeben.

### Beispiel 1

Man kocht eine Lösung von 0,9 mol 2-Chlormethyl-4-phenyl-pyridin-hydrochlorid ("A") [F. 174-1760; erhältlich durch Reduktion von 4-Phenyl-pyridin-2-carbonsäure mit LiAIH₄ zu 2-Hydroxymethyl-4-phenyl- pyridin (F. 183-1840) und Reaktion mit SOCI_{2]}, 1 mol 2-Aminomethyl-1,4-benzodioxan und 5 ml Triethylamin in 100 ml Acetonitril 12 Stunden, arbeitet wie üblich auf und erhält N-(1,4-Benzodioxan-2-yl-methyl)-N-(4-phenyl-2-pyridyl)-amin (Dihydrochlorid), F. 237-2380.

Analog erhält man durch Umsetzung von "A"
mit N-(1,4-Benzodioxan-2-yl)-methyl-N-methyl-amin: N-(2,4-Benzodioxan-2-yl-methyl)-N-(4-phenyl-2-pyridyl)-N-methyl-amin;
mit N-[2-(1,4-Benzodioxan-2-yl)-ethyl]-amin: N-(4-Phenyl-2-pyridyl-methyl)-N-[2-(1,4-benzodioxan-2-yl)-ethyl]-amin;
mit N-[2-(1,4-Benzodioxan-2-yl)-ethyl]-N-methyl-amin: N-(4-Phenyl-2-pyridyl-methyl)-N-[2-(1,4-benzodioxan-2-yl)-ethyl]-N-methyl-amin;
mit N-(1,4-Benzodioxan-2-yl)-methyl-N-ethyl-amin: N-(4-Phenyl-2-pyridyl-methyl)-N-(1,4-benzodioxan-2-yl-methyl)-N-ethyl-amin;
mit N-[2-(1,4-Benzodioxan-2-yl)-ethyl]-N-propyl-amin: N-(4-Phenyl-2-pyridyl-methyl)-N-[2-(1,4-benzodioxan-2-yl)-ethyl]-N-propyl-amin;
mit N-[2-(1,4-Benzodioxan-2-yl)-ethyl]-N-isopropyl-amin: N-(4-Phenyl-2-pyridyl-methyl)-N-[2-(1,4-benzodioxan-2-yl)-ethyl]-N-isopropyl-amin.

### Beispiel 2

Eine Mischung von 2,1 g 3-p-Fluorphenyl-5-aminomethyl-pyridin [erhältlich durch LiAIH₄-Reduktion von 3-p-Fluorphenyl-5-cyan-pyridin] und 1,6 g 2-Chlormethyl-1,4-benzodioxan ("B"), gelöst in 40 ml Wasser und 40 ml Aceton, wird 16 Stunden gekocht und wie üblich aufgearbeitet. Man erhält N-(3-p-Fluorphenyl-5-pyridyl-methyl)-N-(1,4-benzodioxan-2-yl-methyl)-amin; Hydrochlorid, F. 221-222°.

Analog erhält man durch Umsetzung von "B"
mit 3-p-Methoxyphenyl-5-aminomethyl-pyridin: N-(3-p-Methoxyphenyl-5-pyridyl-methyl)-N-(1,4-benzodioxan-2-yl-methyl)-amin, Dihydrochlorid; F. 228-230°;
mit 3-Phenyl-5-aminomethyl-pyridin: N-(3-Phenyl-5-pyridyl-methyl)-N-(1,4-benzodioxan-2-yl-methyl)-amin, Dihydrochlorid; F. 230-233 °;
mit 3-p-Cyanphenyl-5-aminomethyl-pyridin: N-(3-p-Cyanphenyl-5-pyridyl-methyl)-N-(1,4-benzodioxan-2-yl-methyl)-amin;
mit 3-p-Chlorphenyl-5-aminomethyl-pyridin: N-(3-p-Chlorphenyl)-5-pyridyl-methyl)-N-(1,4-benzodioxan-2-yl-methyl)-amin;
mit 3-p-Bromphenyl-5-aminomethyl-pyridin: N-(3-p-Bromphenyl-5-pyridyl-methyl)-N-(1,4-benzodioxan-2-yl-methyl)-amin;
mit 3-(2,4-Dimethoxyphenyl)-5-aminomethyl-pyridin: N-[3-(2,4-Dimethoxyphenyl)-5-pyridyl-methyl]-N-(1,4-benzo-dioxan-2-yl-methyl)-amin;
mit 3-(2,4-Dichlorphenyl)-5-aminomethyl-pyridin: N-[3-(2,4-Dichlorphenyl)-5-pyridyl-methyl]-N-(1,4-benzodioxan-2-yl-methyl)-amin;
mit 3-p-Methylphenyl-5-aminomethyl-pyridin: N-(3-p-Methylphenyl-5-pyridyl-methyl)-N-(1,4-benzodioxan-2-yl-methyl)-amin;
mit 3-m-Methylphenyl-5-aminomethyl-pyridin: N-(3-m-Methylphenyl-5-pyridyl-methyl)-N-(1,4-benzodioxan-2-yl-methyl)-amin;
mit 3-p-Ethoxyphenyl-5-aminomethyl-pyridin: N-(3-p-Ethoxyphenyl-5-pyridyl-methyl)-N-(1,4-benzodioxan-2-yl-methyl)-amin;
mit 3-(3,4-Dimethoxyphenyl)-5-aminomethyl-pyridin: N-[3-(3,4-Dimethoxyphenyl)-5-pyridyl -methyl]-N-(1,4-benzodioxan-2-yl-methyl)-amin.

### Beispiel 3

Zu einer Suspension von 0,6 g Diisobutylaluminiumhydrid (DIBAH) in 20 ml THF wird unter Rühren in einer N₂-Atmosphäre bei 20 eine Lösung von N-(3-p-Methoxyphenyl-5-pyridyl-methyl)-N-(1,4-benzodio- xan-2-yl-methyl)-amin in 40 ml THF zugetropft. Man rührt 1 Std. bei 20_{°}, zersetzt mit verdünnter Natronlauge, filtriert, arbeitet das Filtrat wie üblich auf und erhält N-(3-p-Hydroxyphenyl-5-pyridyl-methyl)-N-(1,4-benzodioxan-2-yl-methyl)amin.

### Beispiel 4

Analog Beispiel 3 erhält man ausgehend von N-[3-(2,4-Dimethoxy-phenyl)-5-pyridyl -methyl]-N-(1,4-benzodioxan-2-yl-methyl)-amin durch Umsetzung mit 1,1 g DIBAH und üblicher Aufarbeitung N-[3-(2,4-Dihydroxyphenyl)-5-pyridyl -methyl]-N-(1,4-benzodioxan-2-yl-methyl)-amin.

### Beispiel 5

Eine Lösung von 2,8 g N-[3-(p-Methoxyphenyl)-5-pyridyl-methyl]-N-(1,4-benzodioxan-2-yl-methyl)-amin (F. 228-2300) in 40 ml Dimethylformamid wird unter Eiskühlung mit 0,3 g NaH versetzt und 1 Stunde gerührt. Anschließend fügt man 1,5 ml Ethyliodid hinzu, rührt weitere 2 Stunden und erhält nach üblicher Aufarbeitung N-[3-(p-Methoxyphenyl)-5-pyridyl-methyl]-N-(1,4-benzodioxan-2-yl-methyl)-N-ethyl-amin.

Analog erhält man durch Alkylierung der entsprechenden sekundären Amine der Formel I:
N-(4-Phenyl-2-pyridyl-methyl)-N-(1,4-benzodioxan-2-yl-methyl)-N-ethyl-amin;
N-(4-p-Methylphenyl-2-pyridyl-methyl)-N-(1,4-benzodioxan-2-yl-methyl)-N-ethyl-amin;
N-(4-Phenyl-2-pyridyl-methyl)-N-[2-(1,4-benzodioxan-2-yl)-ethyl]-N-ethyl-amin;
N-(4-p-Methylphenyl-2-pyridyl-methyl)-N-[2-(1,4-benzodioxan-2-y1)-ethyl]-N-methyl-amin;
N-(4-m-Methoxyphenyl-2-pyridyl-methyl)-N-(1,4-benzodioxan-2-yl-methyl)-N-ethyl-amin;
N-(4-p-Methylphenyl-2-pyridyl-methyl)-N-[2-(1,4-benzodioxan-2-yl)-ethyl]-N-propyl-amin;
N-(4-p-Methoxyphenyl-2-Pyridyl-methyl)-N-[2-(1,4-benzodioxan-2-yl)-ethyl]-N-isopropyl-amin;
N-(3-p-Methoxyphenyl-5-pyridyl-methyl)-N-(1,4-benzodioxan-2-yl-methyl)-N-isopropyl-amin;
N-(3-Phenyl-5-pyridyl)-methyl)-N-(1,4-benzodioxan-2-yl-methyl)-N-methyl-amin;
N-(3-p-Cyanphenyl-5-pyridyl-methyl)-N-(1,4-benzodioxan-2-yl-methyl)-N-ethyl-amin;
N-(3-p-Chlorphenyl-5-pyridyl-methyl)-N-(1,4-benzodioxan-2-yl-methyl)-N-ethyl-amin;
N-(3-p-Bromphenyl-5-pyridyl-methyl)-N-(1,4-benzodioxan-2-yl-methyl)-N-methyl-amin;
N-[3-(2,4-Dimethoxyphenyl)-5-pyridyl-methyl]-N-(1,4-benzodioxan-2-yl-methyl)-N-ethyl-amin;
N-(3-p-Fluorphenyl-5-pyridyl-methyl)-N-(1,4-benzodioxan-2-yl-methyl)-N-methyl-amin, Rf = 0,3 (Ethylacetat);
N-[3-(2,4-Dichlorphenyl)-5-pyridyl-methyl]-N-(1,4-benzodioxan-2-yl-methyl)-N-propyl-amin;
N-(3-p-Methylphenyl-5-pyridyl-methyl)-N-(1,4-benzodioxan-2-yl-methyl)-N-isopropyl-amin;
N-(3-m-Methylphenyl-5-pyridyl-methyl)-N-(1,4-benzodioxan-2-yl-methyl)-N-isopropyl-amin;
N-(3-p-Ethoxyphenyl-5-pyridyl-methyl)-N-(1,4-benzodioxan-2-yl-methyl)-N-isopropyl-amin;
N-[3-(3,4-Dimethoxyphenyl)-5-pyridyl-methyl]-N-(1,4-benzodioxan-2-yl-methyl)-N-butyl-amin;
N-[3-(2,4-Hydroxyphenyl)-5-pyridyl-methyl]-N-(1,4-benzodioxan-2-yl-methyl)-N-butyl-amin.

### Beispiel 6

Man löst 0,1 mol (±)-N-(3-p-Fluorphenyl-5-pyridyl-methyl)-N-(1,4-benzodioxan-2-yl-methyl)-amin in einer Ethylacetat/Ethanol-Mischung (400 ml; 3:1) bei 50_{°} und fügt 0,05 mol L-(+)-Mandelsäure, gelöst in 350 ml des gleichen Lösungsmittelgemisches, hinzu. Anschließend kühlt man auf Raumtemperatur ab und läßt die Reaktionsmischung 64 Stunden zur Kristallisation stehen. Die entstandenen Kristalle werden abgetrennt und zur weiteren Reinigung aus Ethylacetat/Ethanol (3:1) umkristallisiert. Man erhält (+)-N-[3-(p-Fluorphenyl)-pyridyl-methyl)-N-(1,4-benzodioxan-2-yl-methyl)-amin-L-(+)-mandelat. Durch anschließende Behandlung mit Triethylamin erhält man R-(+)-N-(3-p-Fluorphenyl-5-pyridyl -methyl)-N-(1,4-benzodioxan-2-yl-methyl)-amin, Rf 0,29 (Aceton); [α]20 D =33,3 (Methanol).

### Beispiel 7

Die bei der Kristallisation nach Beispiel 6 erhaltneen Mutterlaugen werden eingeengt, das Konzentrat mit 25 ml Methanol versetzt und auf 50 °erwärmt. Anschließend fügt man 0,27 mol D-(-)-Dihenzoylweinsäu- re, gelöst in 40 ml Methanol, hinzu und kühlt zur Kristallisation auf 0 ° ab. Man erhält nach Abtrennung der Kristalle und zusätzlicher Reinigung durch Umkristallisieren aus Methanol (-)-N-(3-p-Flurophenyl)-5-pyridyl - methyl)-N-(1,4-benzodioxan-2-yl-methyl-amin-D-(-)-dibenzoyltartrat. Durch anschließende Behandlung mit Triethylamin erhält man S-(-)-N-(3-p-Fluorphenyl-5-pyridyl -methyl)-N-(1,4-benzodioxan-2-yl-methyl)-amin, Rf 0,29 (Aceton); [α]20 D = -28,6 °(Methanol).

Die nachstehenden Beispiele betreffen pharmazeutische Zubereitungen, die 1,4-Benzodioxanderivate der Formel 1 oder ihre Säureadditionssalze enthalten:

### Beispiel A: Tabletten

Ein Gemisch von 1 kg N-(3-Phenyl-5-pyridyl-methyl)-N-1,4-benzodioxan-2-yl-methyl)-amin, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel B: Dragees

Analog Beispiel A werden Tabletten gepreßt, die anschließend in üblicher Weise mit einerm Überzug aus Saccharose, Kartoffelstärke, Talk, Tragant und Farbstoff Überzogen werden.

### Beispiel C: Kapseln

2 kg N-(3-p-Methoxyphenyl-5-pyridyl-methyl)-N-(1,4-benzodioxan-2-yl-methyl)-amin werden in üblicher Weise in Hartgelatinekapseln gefüllt, so daß jede Kapsel 20 mg des Wirkstoffs enthält.

### Beispiel D: Ampullen

Eine Lösung von 1 kg N-(4-Phenyl-2-pyridyl-methyl)-N-(1,4-benzodioxan-2-yl-methyl)-amin-dihydrochlorid in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche

1. 1,4-Benzodioxanderivate der Formel I 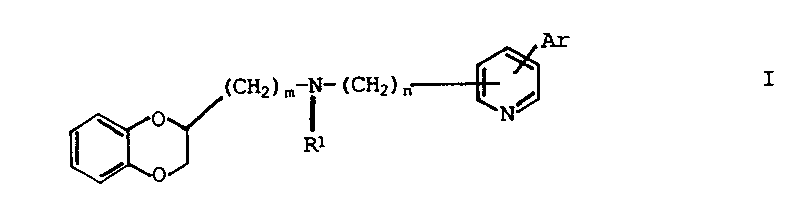 worin
R¹ H oder A,
Ar einen unsubstituierten oder einen ein- oder zweifach durch A, F, Cl, Br, I, CN, OH und/oder OA oder durch eine Methylendioxygruppe substituierten Phenylrest,
A Alkyl mit 1-6 C-Atomen und
m und n jeweils unabhängig voneinander 1 oder 2
bedeuten,
sowie deren Salze. 2.
a) N-(1,4-Benzodioxan-2-yl-methyl)-N-(4-phenyl-2-pyridyl-methyl)-amin und dessen Säureadditionssalze;
b) N-(1,4-Benzodioxan-2-yl-methyl)-N-(5-phenyl-3-pyridyl-methyl)-amin und dessen Säureadditionssalze;
c) N-(1,4-Benzodioxan-2-yl-methyl)-N-(5-p-flurophenyl-3-pyridyl-methyl)-amin und dessen Säureadditionssalze;
d) N-(1,4-Benzodioxan-2-yl-methyl)-N-(5-p-methoxyphenyl-3-pyridyl-methyl)-amin und dessen Säureadditionssalze.

3. Verfahren zur Herstellung von 1,4-Benzodioxanderivaten der Formel I nach Anspruch 1 sowie von deren Salzen, dadurch gekennzeichnet, daß man eine Verbindung der Formel II 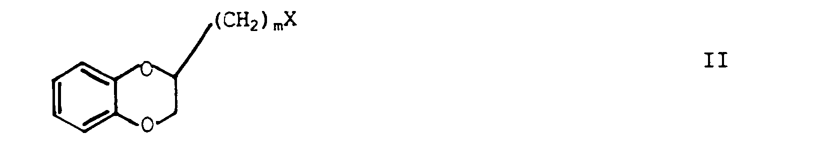 worin m die angegebene Bedeutung hat und
X Cl, Br, I, OH oder eine reaktionsfähige, funktionell abgewandelte OH-Gruppe bedeutet, mit einer Verbindung der Formel III 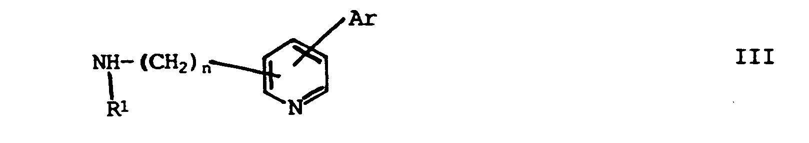 worin
R¹, Ar und n die angegebenen Bedeutungen haben, umsetzt,
oder daß man eine Verbindung der Formel IV 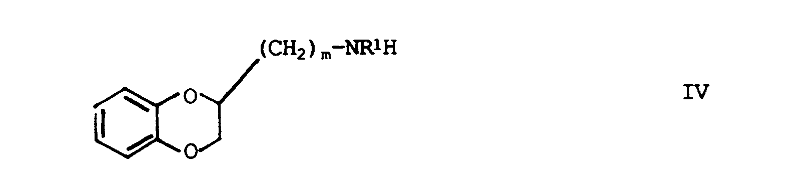 worin
R¹ und m die angegebenen Bedeutungen haben, mit einer Verbindung der Formel V 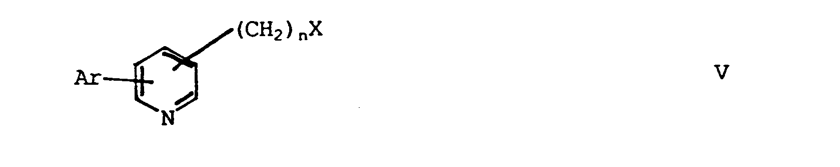 worin
Ar, n und X die angegebenen Bedeutungen haben, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzlich C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe (n) enthält, mit einem solvolysierenden Mittel behandelt,
und/oder daß man gegebenenfalls ein sekundäres Amin der Formel I (R¹ = H) durch Alkylierung in ein tertiäres Amin der Formel I (R¹ = A) umwandelt und/oder daß man eine Gruppe Ar in eine andere Gruppe Ar und/oder daß man eine erhaltene Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel 1 nach Patentanspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel 1 nach Patentanspruch 1 und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

7. Verwendung von Verbindungen der Formel I nach Patentanspruch 1 oder von deren physiologisch unbedenklichen Salzen bei der Bekämpfung von Krankheiten.
